(19) Europäisches Patentamt
European Patent Office
Office européen des brevets

(11) Publication number: **0 370 160 B1**

(12) **EUROPEAN PATENT SPECIFICATION**

(45) Date of publication of patent specification: **04.05.94**

(51) Int. Cl.[5]: **C12N 1/16**, A21D 8/04, //(C12N1/16,C12R1:645)

(21) Application number: **89102818.5**

(22) Date of filing: **18.02.89**

(54) **Method for preparing bakers' yeast.**

(30) Priority: **24.11.88 JP 294508/88**

(43) Date of publication of application:
**30.05.90 Bulletin 90/22**

(45) Publication of the grant of the patent:
**04.05.94 Bulletin 94/18**

(84) Designated Contracting States:
**DE FR GB IT**

(56) References cited:
**EP-A- 0 197 497**
**FR-A- 1 599 865**

**CEREAL CHEMISTRY. vol. 64, no. 4, August 1987, MINNEAPOLIS US pages 269 - 275;HINO, A. et al.: "New freeze-tolerant yeast for frozen dough preparations"**

(73) Proprietor: **Director of National Food Research Institute Ministry of Agriculture Forestry and Fisheries**
**2-1-2, Kannondai**
**Yatabe-machi**
**Tsukuba-gun Ibaraki-ken(JP)**

Proprietor: **YAMAZAKI BAKING CO., LTD**
**2-4, 3-chome, Iwamoto-cho**
**Chiyoda-ku Tokyo(JP)**

(72) Inventor: **Takano, Hiroyuki**
**702-201, 2-chome**
**Azuma**
**Tsukuba-shi Ibaraki-ken(JP)**
Inventor: **Hino, Akihiro**
**804-303, 1-chome**
**Takezono**
**Tsukuba-shi Ibaraki-ken(JP)**
Inventor: **Yamada, Yoshitaka**
**2-13-103, 2-chome**
**Kasuga-cho**
**Iruma-shi Saitama-ken(JP)**
Inventor: **Yoshikawa, Shigenori**
**6-1-611, 4-chome**
**Shiratori**
**Katsuhika-ku Tokyo(JP)**

(74) Representative: **Türk, Gille, Hrabal, Leifert**
**Brucknerstrasse 20**
**D-40593 Düsseldorf (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

### BACKGROUND OF THE INVENTION

The present invention relates to a method for preparing bakers' yeast and, more particularly, to a method for preparing bakers' yeast able to increase bread making ability and make much use of freeze tolerance.

It is believed that the use of said yeast makes remarkable progress in the frozen dough baking method and makes it possible to provide fresh-baked bread to consumers.

The present invention relates to a method for preparing Kluyveromyces thermotolerans FRI-501 (FERM BP-2243) as the bakers' yeast, and makes much use of satisfactory bread making ability and freeze tolerance, which said yeast possesses but could not be extracted by conventional techniques, by improving the method for cultivating said yeast.

In general, bakers' yeast is prepared by cultivating seed yeast and, then, carrying out the large scale final cultivation with the use of the obtained seed yeast. In the cultivation, clarified molasses obtained by fining waste molasses is widely used as a main medium substrate, with which nitrogen and phosphorus are used as complementary substrates. Urea, ammonium sulfate, ammonia, etc. are used as the nitrogen sources and calcium perphosphate, phosphoric acid, phosphates, etc. as the phosphorus sources. For the large scale final cultivation, use is made of the socalled exponetial fed-batch method in which a molasses liquid is exponentially fed according to the predetermined amount of cultivation, since yeast is exponentially propagated under ideal conditions. Referring in detail to the cultivation conditions, the optimum amount of sugar to be fed for the cultivation of yeast, i.e., the amount of sugar to be fed to one gram of wet yeast (calculated as 70% moisture) per hour (hereinafter called the feed rate) is predetermined, and molasses is fed in an amount corresponding to the amount of sugar culculated by using the feed rate. Conventionally, the cultivation of bakers' yeast is carried out at the basic feed rate of 0.16 to 0.18. The feeding of nitrogen and phosphorus is performed in parallel with the feeding of molasses. Alternatively, they may be added to an initial medium in the required amounts with the predetermined final amount of yeast in mind. Of importance is here the weight ratio of nitrogen and phosphorous to be added to the amount of sugar, wherein the ratio of sugar to nitrogen or phosphorous is 100 to 2.5-5.0 and 100 to 0.3-0.5, respectively.

The cultivation temperature is ordinarily regulated in a range of 25 to 35 °C, and the pH should be adjusted depending upon the type of nitrogen to be added and be generally maintained in a range of 4 to 6. Additionally, oxygen is supplied in the amount required for the aerobic cultivation of yeast and to avoid any conversion to ethanol fermentation due to the Pasteur effect. Conventionally, such cultivation is continued for 8 to 16 hours, followed by 1 to 6-hour culture for maturation. Such maturation culture relies upon the socalled nitrogen starvation culture method in which molasses without nitrogen is supplied at the basic feed rate of approximately 0.04, whereby the preservability (or storability) of yeast is improved.

However, it has now been found that when yeast FRI-501 strains excelling extremely in freeze tolerance, as disclosed in Japanese Patent Kokai Koho 61-254186, are prepared by the aforesaid conventional technique on an industrial scale, the resulting yeast is poor in fermentation ability. It should be noted that the yeast strain Saccharomyces cerevisieae IFTY-2 (FRI-501) mentioned in the above document and Kluyveromyces thermotolerans FRI-501 (FERM BP-2243) are the same. For that reason, there is left a problem to be solved about the culture method of said yeast in order to make use of yeast FRI-501 strain for bread-making, inter alia, frozen dough baking. This has led to investigations on the method for preparing said yeast for the purpose of making much of the bread making ability and freeze tolerance thereof, and to a discovery that yeast of very high quality can be obtained from said yeast by a method unconceivable in the conventional preparation of bakers' yeast whatsoever.

### SUMMARY OF THE INVENTION

More specifically, the present invention provides a method for preparing bakers' yeast with the use of freeze tolerance yeast Kluyveromyces thermotolerans FRI-501 (FERM BP-2243), wherein at the time of the starvation culture of said yeast, a carbon source is added at a concentration accounting for 25 to 200% of the weight of yeast cells, and the amount of aeration is reduced.

### BRIEF DESCRIPTION OF THE DRAWING

Figure 1 is a graphical view showing a difference in fermentation ability when the amount of the sugar added is varied after the feeding culture in Example 3, with the amount (%) of the sugar added to the amount of yeast cells as ordinate and the liquid fermentation ability (ml) as abscissa.

DETAILED DESCRIPTION OF THE INVENTION

The present invention relates to a method for preparing bakers' yeast with the use of freeze tolerant Kluyveromyces thermotolerans FRI-501 (FERM BP-2243), wherein a large scale final cultivation with the use of seed yeast is carried out according to an exponential fed-batch method before the starvation culture of said yeast characterized in that at a time of a starvation culture of said yeast, carbon source at a concentration accounting for 25 to 200 % of the weight of yeast cells is added and the amount of aeration is reduced.

In the case of ordinary bakers' yeast, it has been known that when cultivating it at a high sugar concentration, its conversion to ethanol fermentation is apt to take place due to the Crabtree effect, whereby bakers' yeast of decreased fermentation ability and low quality is obtained in considerably limited yields of yeast. According to the present invention, bakers' yeast of extremely high fermentation ability can be obtained by cultivating at rather high sugar concentrations. Such a discovery underlies the method for preparing bakers' yeast according to the present invention. The most important point of the present invention is that the aforesaid exponential feeding culture (as conventionally applied) is carried out until the predetermind concentration of yeast cells is reached in the large scale final cultivation, and the next starvation culture is carried out with rapid addition of an excess of sugar simultaneously with a reduction in the amount of aeration for several hours to collect yeast cells.

More specifically, the exponential feeding culture is carried out until the amount of yeast cells (hereinafter referring to the weight of wet yeast) accounts for 40 to 150 g per liter of a culture solution, and molasses containing 25 to 200%, preferably 50 to 150% of sugar with respect to the amount of yeast cells is rapidly added upon the predetermined concentration of yeast cells being reached. The addition of sugar in an amount exceeding 200% is impractical with economical considerations and the volume of a culture vessel in mind. Besides, any effect corresponding to such a large amount cannot be obtained. On the other hand, no satisfactory effect is attained by the addition of less than 25% of sugar. With the sugar added in the aforesaid proportion, the starvation culture is continued for 1 to 6 hours. It is noted that the aeration-agitation conditions are 100 to 400 rpm and at most 4 l/min. The fermentation ability of the yeast obtained by this method is much more improved over that achieved by the conventional method for preparing bakers' yeast. In addition, the yeast obtained according to the present invention shows improved freeze tolerance in the frozen dough baking method. Thus, excellent bread, especially in flavor and volume, can be obtained from dough after thawing.

The frozen dough baking method has already been put to practical use. In the conventional frozen dough method, however, dough has been frozen without fermentation prior to freezing, because the yeast in the dough subjected to fermentation loses its fermentation ability after thawing. For that reason, a certain limitation is imposed upon the types of bread made by this method. Further, bread obtained by thawing and baking is lacking in the flavor peculiar to bread.

However, the yeast obtained according to the present invention is higher than that obtained by the conventional fermentation method in terms of fermentation power and is also improved thereover in terms of refrigeration resistance. Thus, even after thawing, it retains fermentation power so satisfactory that the bread obtained by thawing and baking is of excellent quality.

By using the bakers' yeast obtained according to the present invention, it is therefore possible to apply the refrigerated dough baking method to much more types of bread than now available, thereby achieving remarkable progress in the refrigerated dough baking method and making it possible to provide fresh-baked bread of high quality to consumers.

The present invention will now be explained with reference to the following examples.

Example 1

The bakers' yeast products obtained from Kluyveromyces thermotolerans FRI-501 strain (FERM BP-2243) by the present and conventional methods were compared with each other in terms of quality. Used to this end was a jar fermentor having a total volume of 15 ℓ. For the exponential feeding culture under the aeration-agitation conditions of 600 rpm and 6 ℓ/min., 0.03% of $MgSO_4 \cdot 7H_2O$ were previously added to an initial medium at a culture temperature of 30 °C and pH 5.2, and the feeding medium used contained 30% of molasses and $CO(NH_2)_2$ and $KH_2PO_4$ which were added thereto in amounts calculated to be 5% as nitrogen and 0.3% as phosphorus, respectively. According to the present method, the exponential feeding culture was carried out under such conditions for 10 hours. Afterwards, molasses containing 125% of sugar with respect to the weight of yeast cells was rapidly added for two-hour culture with the aeration-agitation conditions being changed to 200 rpm and 1 ℓ/min. According to the conventional method, on the other hand, the exponential feeding culture was performed for 10 hours, followed by the nitrogen starvation culture (under the aeration-agitation conditions of 600 rpm and 6 ℓ/min.) at the feed rate of 0.04.

The results are set forth in Table 1, from which it is found that the bakers' yeast obtained by the present invention is much more improved in terms of fermentation ability, and makes a difference with the conventional yeast in terms of the nitrogen content of yeast cells.

Table 1

| | Fermentation Ability (mℓ)*1 | | Nitrogen Content of Yeast Cells (%)*2 |
|---|---|---|---|
| | F10 | F40 | |
| Present Method | 280 | 180 | 7.68 |
| Conventional Method | 160 | 110 | 6.87 |

*1: The fermentation ability was determined by the liquid fermentation ability test according to the Japan Yeast Industry Association. F10 and F40 stand for the amounts (mℓ) of carbon dioxide generated over three hours from the Atkins modified media having 10% and 40% sucrose concentrations, respectively.
*2: The nitrogen content was measured by the kjeldal method, and is expressed in an amount with respect to the dry weight.

Example 2

With the yeast cells obtained by the present method, baking tests were carried out according to the frozen dough baking method to compare the present yeast with commercially available yeast for frozen dough in terms of bread making ability and freeze tolerance.

The formulations of dough and the baking steps are set out in the following table.

| Formulation of Dough | | | |
|---|---|---|---|
| | Low-Sugar Dough | Medium-Sugar Dough | High-Sugar Dough |
| Wheat Flour | 100 | 100 | 100 |
| Yeast | 5 | 5 | 5 |
| Yeast Food | 0.1 | 0.1 | 0.1 |
| Sugar | 5 | 15 | 25 |
| Salt | 2 | 1.5 | 1 |
| Shortening | 5 | 5 | 5 |
| Water Fed | 57 | 56 | 52 |

Steps

Mixing Time: L3, M2, 1, L2, M2 and H1 or longer; viz., three minutes at a low speed, two minutes to a medium speed, two minutes at a low speed with the addition of shortening, two minutes at a low speed, and one or longer minutes at a high speed.

Dough Temp.: 27°C

Scaling Weight: 40 g

Freezing Conditions: Rapid freezing at -40°C for 1 hour, following storage in a freezer of -20°C.

Thawing and Proofing Conditions: Thawing at a temperature of 38°C and relative humidity of 80% for 60 minutes, followed by 45-minute proofing for the low- and medium-sugar dough and 55-minute proofing for the high-sugar dough.

Baking Conditions: Baking at 230 °C for 8 minutes.

With the aforesaid formulations and by the aforesaid bread making steps, bans dough was prepared. The low-, medium- and high sugar dough were fermented for 30 minutes, 60 minutes and 90 minutes, respectively, and were thereafter frozen for up to 3 weeks. The products were then subjected to thawing and proofing and baked to compare their volumes. Table 2 shows that the bans prepared with the use of the yeast obtained by the present method are larger in volume than that prepared by the commercially available yeast for frozen dough and that the frozen dough prepared by this method also excels in storability.

Table 2

| | Bans Volume (mℓ) | |
|---|---|---|
| | 1-Week Freezing | 3-Week Freezing |
| FRI-501 | | |
| Low-sugar dough | 222 | 190 |
| Medium-sugar dough | 223 | 206 |
| High-sugar dough | 215 | 217 |
| Commercially available yeast for frozen dough | | |
| Low-sugar dough | 188 | 179 |
| Medium-sugar dough | 159 | 158 |
| High-sugar dough | 196 | 182 |

Example 3

In the method according to the present invention, the influence on the fermentation ability of the amount of the sugar added after the exponential feeding culture was tested at four-stage concentrations of 25%, 50%, 100% and 200% with respect to the weight of yeast cells. The results are set forth in Figure 1, from which it is found that all the present products are much higher than the control in Example 1 in terms of fermentation ability (measured in a similar manner as described in Example 1).

**Claims**

1. A method for preparing bakers' yeast with the use of freeze tolerant Kluyveromyces thermotolerans FRI-501 (FERM BP-2243) wherein a large scale final cultivation with the use of seed yeast is carried out according to an exponential fed-batch method before the starvation culture of said yeast characterized in that at a time of a starvation culture of said yeast, carbon source at a concentration accounting for 25 to 200% of the weight of yeast cells is added ad the amount of aeration is reduced.

2. The method according to Claim 1, wherein an amount of sugar to be fed to one gram of wet yeast (culculated as 70% moisture) per hour is from 0.16 to 0.18 at the time of the large scale final cultivation.

3. The method according to Claim 1, wherein the weight ratio of sugar to nitrogen and phosphorus to be added is 100 to 2.5-5.0 and 100 to 0.3-0.5, respectively.

4. The method according to Claim 1, wherein the large scale final cultivation is carried out until the amount of the yeast cells accounts for 40 to 150 grams per liter of a culture solution.

**Patentansprüche**

1. Verfahren zur Herstellung von Bäckerhefe (Backhefe) unter Verwendung von einfrier-tolerantem Kluyveromyces thermotolerans FRI-501 (FERM BP-2243), bei dem eine Schlußkultivierung in großem Maßstab unter Verwendung von Impf-Hefe nach einer exponentiellen Zuführungs (Fütterungs)-Batch-Methode vor der Mangel(Hunger)kultivierung dieser Hefe durchgeführt wird, dadurch gekennzeichnet, daß zum Zeitpunkt der Mangel(Hunger)kultivierung dieser Hefe eine Kohlenstoffquelle in einer Konzentration zugegeben wird, die 25 bis 200 Gew.-% der Hefezellen entspricht, und daß die Menge der Belüftung herabgesetzt wird.

2. Verfahren nach Anspruch 1, worin die Zuckermenge, die einem Gramm Naßhefe (berechnet mit einem Feuchtigkeitsgehalt von 70 %) pro Stunde zugeführt werden soll, zum Zeitpunkt der großtechnischen Schlußkultivierung 0,16 bis 0,18 beträgt.

3. Verfahren nach Anspruch 1, worin das Gewichtsverhältnis zwischen Zucker und Stickstoff und Phosphor, die zugegeben werden sollen, 100:2,5 bis 5,0 bzw. 100:0,3 bis 0,5 beträgt.

4. Verfahren nach Anspruch 1, worin die großtechnische Schlußkultivierung durchgeführt wird, bis die Menge der Hefezellen 40 bis 150 g pro Liter Kulturlösung beträgt.

**Revendications**

1. Procédé de préparation de levure de boulanger à l'aide de la levure Kluyveromyces thermotolerans FRI-501 (FERM BP-2243), tolérante à la congélation, dans laquelle on effectue une culture finale à grande échelle à l'aide de germes de levure, selon un procédé par lots à charge exponentielle, avant la culture par carence de ladite levure, caractérisé en ce que, au moment de la culture par carence de ladite levure, on ajoute une source de carbone à une concentration représentant 25 à 200% du poids des cellules de levure et on réduit le taux d'aération.

2. Procédé selon la revendication 1, dans lequel la quantité de sucre chargé par gramme de levure humide (calculée à 70% d'humidité) par heure est de 0,16 à 0,18 au moment de la culture finale à grande échelle.

3. Procédé selon la revendication 1, dans lequel le rapport pondéral du sucre à l'azote et au phosphore à ajouter est respectivement de 100 à 2,5-5,0 et de 100 à 0,3-0,5.

4. Procédé selon la revendication 1, dans lequel la culture finale à grande échelle est réalisée jusqu'à ce que la quantité de cellules de levure représente 40 à 150 grammes par litre de solution de culture.

Fig. 1

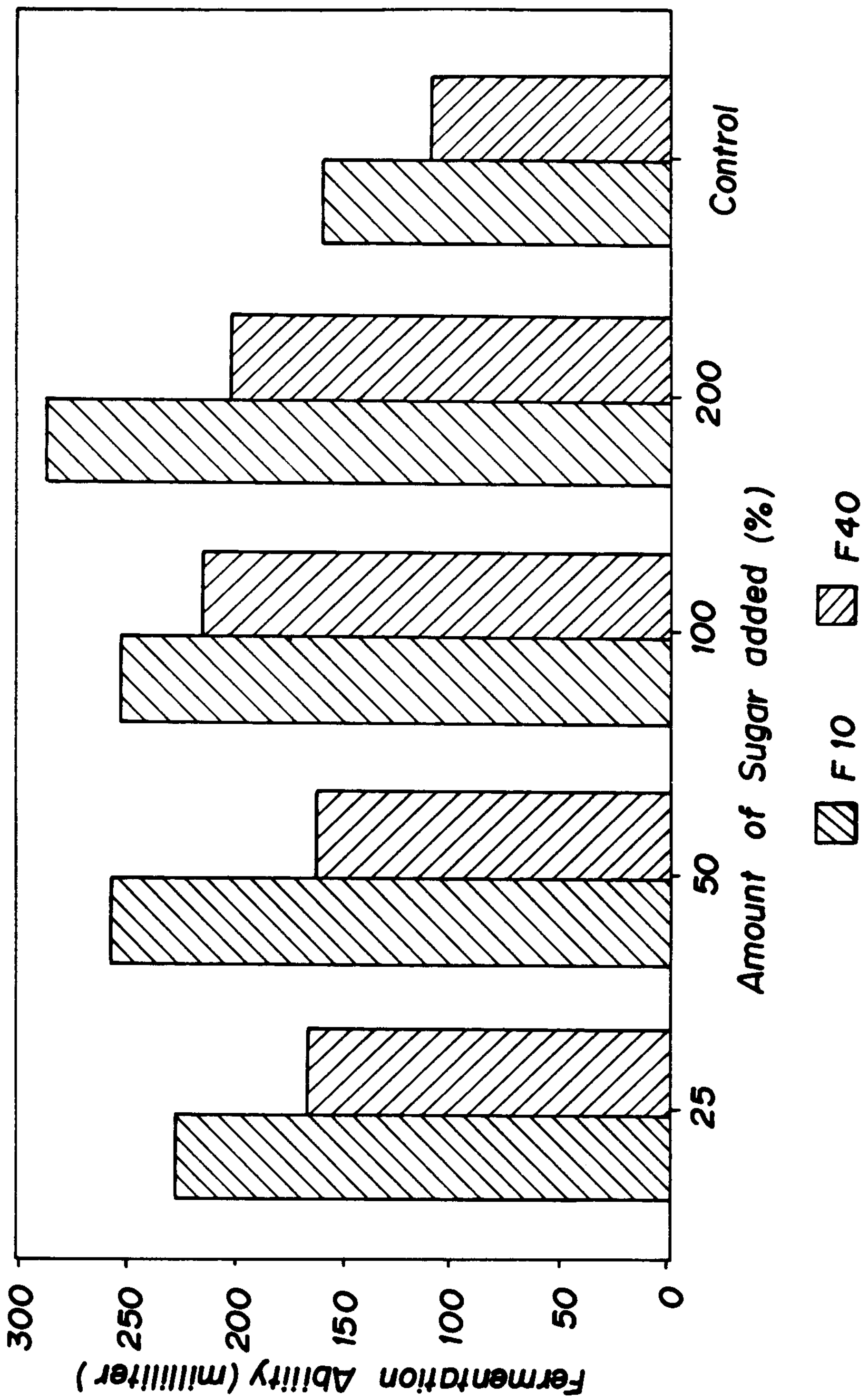
Fermentation Ability (milliliter)
300
250
200
150
100
50
0
25
50
100
200
Control
Amount of Sugar added (%)
F 10
F40